# EUROPÄISCHE PATENTANMELDUNG

(11) **EP 3 189 829 A1**
(43) Veröffentlichungstag der Anmeldung: **12.07.2017**
(21) Anmeldenummer: 16150743.9
(22) Anmeldetag: 11.01.2016
(51) Int. Cl.: A61K 9/00, A61K 45/06, A61Q 5/00, A61K 8/19, A61K 33/00, A61K 33/06, A61P 17/08

(54) **MITTEL MIT ANTI-MYKOTISCHER WIRKUNG**

(71) Anmelder: Justus-Liebig-Universität Gießen, 35390 Gießen (DE)
(72) Erfinder: MAYSER, Peter, 35444 Biebertal (DE)
(74) Vertreter: Stumpf, Peter

(57) **Zusammenfassung**

Gegenstand der Erfindung ist ein Mittel umfassend mindestens ein Alkali- oder Erdalkalisalz zur Prophylaxe und Therapie von Erkrankungen und kosmetischen Problemen bei Menschen und Tieren, welche unter Beteiligung von Malassezia-Hefen verursacht oder aufrechterhalten werden.

## Beschreibung

Die vorliegende Erfindung betrifft Mittel umfassend mindestens ein Alkali- oder Erdalkalisalz zur Prophylaxe und Therapie von Erkrankungen und kosmetischen Problemen bei Menschen und Tieren, welche unter Beteiligung von Malassezia-Hefen verursacht werden. Solche Malassezia-assoziierten Erkrankungen und kosmetische Probleme sind beispielsweise aber nicht abschließend Pityriasis versicolor, seborrhoisches Ekzem (SE), Head-Neck-Dermatitis, Dandruff (erhöhte Schuppenbildung der Kopfhaut, Kopfschuppen), atopische Dermatitis (atopisches Ekzem), Psoriasis, Papillomatose, Onychomykose, akantholytische Dermatose, follikuläre Hyperkeratose, Malassezia-Dermatitis bei Hunden und Katzen und Otitis externa bei Hunden und Katzen.

Insbesondere betrifft die vorliegende Erfindung Mittel umfassend Lithium-, Natrium-, Kalium-, Magnesium-, Calcium-, Beryllium-, Barium- und Strontium-Succinat oder Lithium-, Natrium-, Kalium-, Magnesium-, Calcium-, Beryllium-, Barium- und Strontium-Gluconat als Wirkstoffe zur Behandlung von seborrhoischem Ekzem (SE) oder Kopfschuppen (Dandruff).

### Abkürzungen und Definitionen

**Antimykotikum (Plural: Antimykotika):** Stoff (Stoffe) zur Prophylaxe und Therapie von Pilzinfektionen (Mykosen) bei Menschen und Tieren. Ein Antimykotikum kann topisch (d.h. lokal am Ort der Pilzinfektion) angewendet werden, oder systemisch (d.h. es wird über den Blut- oder Lymphkreislauf im ganzen Körper verteilt).

**Bernsteinsäure:** Succinylsäure, Butandisäure; Summenformel C₄H₆O₄. Succinate (Sukzinate) sind die Salze der Bernsteinsäure.

**Dandruff:** Kopfschuppen, erhöhte Anzahl von abgestorbene Hautschuppen im Kopfbereich. Malassezia-Hefen sind ein Auslöser für Dandruff.

**FFA:** Freie Fettsäure/n (free fatty acid).

**Gluconsäure:** Glukonsäure, Dextronsäure; Summenformel C₆H₁₂O₇. Gluconate (Glukonate) sind die Salze der Gluconsäure.

**Malassezia**-**assoziierte Erkrankung oder kosmetisches Problem:** Erkrankung oder kosmetisches Problem bei Menschen oder Tieren, welche auf Grund einer Infektion mit Malassezia-Hefen besteht oder aufrechterhalten wird. Beispiele dafür sind Pityriasis versicolor, seborrhoisches Ekzem (SE), Head-Neck-Dermatitis, Dandruff (erhöhte Schuppenbildung der Kopfhaut, Kopfschuppen), atopische Dermatitis (atopisches Ekzem), Psoriasis, Papillomatose, Onychomykose, akantholytische Dermatose, follikuläre Hyperkeratose, Malassezia-Dermatitis bei Hunden und Katzen und Otitis externa bei Hunden und Katzen.

**Mykose:** Pilzinfektion bei Menschen und Tieren, beispielsweise Infektion mit Hefen wie z.B. Malassezia- oder Candida-Hefen.

**Seborrhoisches Ekzem (SE):** Seborrhoische Dermatitis, Morbus Unna; Hautausschlag (Ekzem) besonders auf der Kopfhaut und im Gesicht, meist mit Schuppungen verbunden.

### Beschreibung des allgemeinen Gebietes der Erfindung

Malassezia-Hefen können verschiedene Krankheiten verursachen. Lipophile Malassezia-Hefen verursachen unter anderem das seborrhoische Ekzem (SE) oder Kopfschuppen (Dandruff). Bei den bekannten Antimykotika gegen Malassezia-Hefen gibt es Resistenzen, außerdem sind sie umweltgiftig. Es besteht daher ein Bedarf an ungiftigen Wirkstoffen, mit denen insbesondere die lipophilen Malassezia-Hefen auf der Haut von betroffenen Patienten reduziert werden können, ohne dass die Malassezia-Hefen Resistenzen dagegen entwickeln.

Lipophile Malassezia-Hefen gewinnen den zum Wachstum und zur Homöostase benötigten Kohlenstoff nicht aus Kohlenhydraten, sondern aus Lipiden. Die lipophilen Malassezia-Hefen hydrolysieren durch malassezia-eigene Exo-Lipasen und Exo-Hydrolasen Lipide und verstoffwechseln die dabei entstehenden freien Fettsäuren, welche eine Kettenlänge von mehr als 12 Kohlenstoffatomen aufweisen.

### Stand der Technik

Es gibt eine Vielzahl von antimikrobiell wirksamen Substanzen, von denen einige als Antimykotika zum Einsatz gegen Pilzinfektionen, beispielsweise Malassezia-Infektionen eingesetzt werden.

Eine bekannte Stoffgruppe, die als Antimykotikum verwendet wird, ist die Gruppe der Azole bzw. Pyrrole. Azole interagieren mit der Ergosterol-Biosynthese der Malassezia-Hefen und reduzieren somit die Gesamtpopulation von Malassezia-Hefen auf der Hautoberfläche. Bekannte antimykotische Wirkstoffe aus der Gruppe der Azole bzw. Pyrrole sind beispielsweise Clotrimazol, Ketoconazol, Bifonazol, Econazol, Isoconazol, Sertaconazol, Miconazol, Itraconazol, Fluconazol, Voriconazol, Posaconazol und Fosfluconazol. Antimykotika aus der Gruppe der Azole bzw. Pyrrole werden bevorzugt bei Pityriasis versicolor, seborrhoischem Ekzem und Head-Neck-Dermatitis eingesetzt. Die bekannten antimykotischen Wirkstoffe aus der Gruppe der Azole bzw. Pyrrole können als Nebenwirkung Leberschäden und Fruchtschäden an Embryo oder Fötus verursachen, insbesondere wenn sie systemisch eingesetzt werden.

Weitere bekannte Antimykotika sind beispielsweise Amphotericin B, Nystatin, Natamycin, Griseofulvin, Caspofungin, Cilofungin, Flucytosin und Ciclopirox. Diese Antimykotika haben teils schwerwiegende Nebenwirkungen, teils sind sie nicht gegen Malassezia-Hefen wirksam und teils sind sie nicht für eine topische Anwendung geeignet.

Weitere antimykotisch wirksame Substanzen sind beispielsweise Zinkpyrithion oder Selendisulfid.

Bei den bekannten Antimykotika kann es zu Resistenzbildungen kommen, einerseits durch die langdauernde therapeutische Anwendung (beispielsweise bei der Verwendung von Azolen bzw. Pyrrolen gegen Malassezia-Hefen als Dauertherapie), andererseits durch den Eintrag dieser Antimykotika in die Umwelt (beispielsweise durch nicht ordnungsgemäß entsorgte Medikamentenrestbestände).

Neben diesen bekannten Antimykotika weisen auch freie kurz- und mittelkettige gesättigte Fettsäuren eine topische antimykotische Wirkung auf. Dies gilt beispielsweise für die Caprinsäure, die Caprylsäure oder die Capronsäure, welche eine antimykotische Wirkung gegen Malassezia- und Candida-Hefen zeigen. Ein Nachteil eines direkten topischen Einsatz dieser Fettsäuren ohne weitere Zusatzstoffe in Pflegeprodukten, Kosmetika oder Lokaltherapeutika zur Therapie und Prophylaxe von hefe-assoziierten Erkrankungen ist der intensive und unangenehme Geruch dieser Fettsäuren. Zusätzlich müsste die freie Säurefunktion dieser Fettsäuren gepuffert werden, was bei der Verwendung als Pflegeprodukt, Kosmetikum oder Lokaltherapeutikum schwierig ist.

Außer Antimykotika werden auch weitere Stoffe zur Prophylaxe und Therapie von Erkrankungen und kosmetischen Problemen, die unter Beteiligung von Malassezia-Hefen verursacht oder aufrechterhalten werden, eingesetzt. Dazu gehören beispielsweise entzündungshemmende Stoffe (z.B. Glukokortikoide, Kortikosteroide), Desinfizienzien oder Calcineurin-Inhibitoren. Diese Stoffe können ebenfalls unerwünschte und teilweise schwerwiegende Nebenwirkungen haben.

### Aufgabe

Die Aufgabe der vorliegenden Erfindung ist es, Mittel zur Verfügung zu stellen, welche zur topischen Therapie und Prophylaxe von Erkrankungen und kosmetischen Problemen verwendet werden können, die unter Beteiligung von Malassezia-Hefen verursacht oder aufrechterhalten werden. Diese Mittel sollen eine selektive antimykotische Wirkung gegen Malassezia-Hefen aufweisen, preiswert, nicht allergen und ungiftig sein, sowie zur topischen Anwendung bei Menschen und Tieren geeignet sein.

### Lösung der Aufgabe

Die erfindungsgemäße Aufgabe wird gelöst durch die Verwendung von mindestens einem Salz eines Alkali- oder Erdalkalimetalles als Wirkstoff in einem Mittel gemäß der Ansprüche.

Der erfinderische Gedanke besteht darin, die für das Wachstum der Malassezia-Hefen essentiellen freien Fettsäuren auf der Haut der betroffenen Patienten zu vermindern, indem diese abgebunden werden. Die Alkali- oder Erdalkalisalze reagieren mit den freien Fettsäuren auf der Haut in einer Säuren-Basen-Reaktion unter Bildung von Alkali- oder Erdalkali-Seifen, die präzipitieren und daher von den Malassezia-Hefen nicht mehr verstoffwechselt werden können.

Das erfindungsgemäße Mittel enthaltend Alkali- und/oder Erdalkalisalze bewirkt keine direkte Abtötung der Malassezia-Hefen, sondern vermindert die Verfügbarkeit der für das Wachstum der Malassezia-Hefen essentiellen freien Fettsäuren, welche durch die Malassezia-eigenen Exo-Lipasen und Exo-Hydrolasen generiert wurden. Durch diese verminderte Verfügbarkeit der freien Fettsäuren wird der Stoffwechsel der Malassezia-Hefen gehemmt und das Wachstum der Malassezia-Hefen verringert.

Alkali- oder Erdalkalisalze, insbesondere solche der Bernsteinsäure (Succinate, Sukzinate) reagieren mit den freien Fettsäuren in einer Säuren-Basen-Reaktion unter Bildung von Alkali- oder Erdalkali-Seifen, die präzipitieren und von den Malassezia-Hefen nicht mehr verstoffwechselt werden können. Aus diesem Ansatz resultiert ein therapeutisch nutzbarer antimykotischer Effekt. Ferner werden diese freien Fettsäuren durch die Verseifung mit Alkali- oder Erdalkalisalzen wasserlöslich und damit leichter von der betroffenen Haut abspülbar. Auch dieser antiseborrhoische Effekt führt dazu, dass die für das Wachstum der Malassezia-Hefen essentiellen freien Fettsäuren auf der Haut verringert werden, wodurch das Wachstum und die Vermehrung der Malassezia-Hefen gehemmt werden.

Auch andere toxische oder entzündungsfördernde Produkte der Aktivität der Malassezia-eigenen Exo-Lipasen und Exo-Hydrolasen, wie beispielsweise Lipidperoxide, werden durch die Verseifung mit Alkali- oder Erdalkalisalzen neutralisiert bzw. inaktiviert.

Am besten geeignet sind Succinate (Sukzinate, Salze der Bernsteinsäure) und Gluconate (Glukonate, Salze der Glukonsäure). Aber auch Kombinationen aus Alkali- und/oder Erdalkalisalzen, ausgewählt aus Lithium-, Natrium-, Kalium-, Magnesium-, Calcium-, Beryllium-, Barium- und Strontium-Succinat und/oder Lithium-, Natrium-, Kalium-, Magnesium-, Calcium-, Beryllium-, Barium- und Strontium-Gluconat zeigen eine gute Wirksamkeit gegen Malassezia-Hefen.

Dieser Wirkmechanismus zur Hemmung der Malassezia-Hefen wird auch durch andere Alkali- und/oder Erdalkalisalze erreicht, beispielsweise durch Lithium-, Natrium-, Kalium-, Magnesium-, Calcium-, Beryllium-, Barium- und Strontium-Citrat oder Lithium-, Natrium-, Kalium-, Magnesium-, Calcium-, Beryllium-, Barium- und Strontium-Malat.

Folgende Kombinationen von Alkali- und/oder Erdalkalisalze sind besonders wirksam zur Hemmung der Malassezia-Hefen:
- Lithium-Succinat mit einem beliebigen Gluconat
- Calcium-Succinat mit einem beliebigen Gluconat
- Magnesium-Citrat mit einem beliebigen Succinat oder Gluconat
- Calcium-Citrat mit einem beliebigen Succinat oder Gluconat

Ebenso sind andere Kombinationen von zwei oder mehreren Alkali- und/oder Erdalkalisalzen (Succinate, Gluconate, Citrate oder Malate) zur Hemmung der Malassezia-Hefen wirksam.

Die erfindungsgemäßen Alkali- oder Erdalkalisalze in einem Mittel nach Anspruch 1 rufen im Kontakt mit Wasser keine wesentlich vom Neutralpunkt bei pH=7 abweichende pH-Wert-Änderung im kontaktieren Wasser hervor. Als besonders wesentliche Abweichung vom Neutralpunkt gelten Abweichungen von mehr als 4 pH-Einheiten, als wesentliche Abweichung gelten Abweichungen von mehr als 3 pH-Einheiten und als unwesentliche und daher besonders geringe Abweichung gelten Abweichungen von 3 oder weniger pH-Einheiten vom Neutralpunkt. Da der physiologische pH-Wert von Haut bei pH=5,5 liegt, umfassen die besten erfindungsgemäßen Ausführungsvarianten Salze bzw. Salzmischungen (Puffersysteme), deren pH-Wert in Lösung 5,5 beträgt. Geeignete Verfahren und Verfahrensbedingungen zur Bestimmung der pH-Wert-Änderung, beispielsweise Konzentration des Salzes bei der Messung, sind dem Fachmann bekannt.

Die in Kontakt mit Wasser durch Alkali- oder Erdalkalisalze hervorgerufene pH-Wertänderung ist im Wesentlichen abhängig von dem im Alkali- bzw. Erdalkalisalz enthaltenen Anion. Besonders geeignete Anionen sind Anionen organischer Säuren bzw. Alkohole mit mindestens einer Säure- bzw. alkoholischer Hydroxylgruppe, wie beispielsweise, aber nicht ausschließlich, Acetat, Lactat, Citrat, Succinat, Valerat, Anionen von langkettigen Fettsäuren (C10 bis C30), Anionen langkettiger Fettalkohole (C8 bis C30), Anionen langkettiger Sulfonsäuren oder Anionen von Schwefelsäure-Monoestern mit langkettigen organischen Resten (C8 bis C30).

Die Verwendung von Alkali- oder Erdalkalisalzen als Wirkstoff in einem Mittel zur Therapie oder Prophylaxe von malassezia-assoziierten Erkrankungen beruht auf folgenden unerwarteten Effekten:
- Die Alkali- oder Erdalkalisalze binden die für das Wachstum der Malassezia-Hefen essentiellen Fettsäuren unter Ausbildung von Seifen.
- Die Alkali- oder Erdalkalisalze binden toxische und entzündliche Metaboliten der Aktivität der malassezia-eigenen Exo-Lipasen und Exo-Hydrolasen wie beispielsweise Lipidperoxide.
- Die Alkali- oder Erdalkalisalze beeinflussen die Aktivität der malassezia-eigenen Exo-Lipasen und Exo-Hydrolasen.

Erfindungsgemäß wird das Mittel umfassend mindestens ein Alkali- oder Erdalkalisalz in topischer Anwendung, beispielsweise als Shampoo, Salbe, Lösung, Lotion, Creme, Schüttelmixtur, Schüttelpinselung, Suspension, Emulsion, Paste, Spray, lipophiles Gel, hydrophiles Gel, Pflaster, Badezusatz oder Schlamm eingesetzt. Diese topischen Anwendungen und deren Herstellung sind dem Fachmann bekannt.

Das erfindungsgemäße Mittel kann zusätzlich pharmazeutisch unschädliche Exzepientien und/oder Additive enthalten, um die pharmazeutische oder kosmetische Anwendung des erfindungsgemäßen Mittels zu verbessern. Diese Exzepientien und/oder Additive sind dem Fachmann bekannt.

Das erfindungsgemäße Mittel kann zusätzlich pharmazeutisch unschädliche Vitamine, UV-Schutz oder Duftstoffe enthalten.

Das erfindungsgemäße Mittel wird verwendet, um ein Medikament oder ein Kosmetikum herzustellen, welche zur Prophylaxe oder Therapie von Erkrankungen oder kosmetischen Problemen bei Menschen oder Tieren eingesetzt werden, welche unter Beteiligung von Malassezia-Hefen verursacht oder aufrechterhalten werden.

Das erfindungsgemäße Mittel kann zur Verstärkung der Wirkung mit anderen aus dem Stand der Technik bekannten Antimykotika, juckreizstillenden Substanzen, Antiphlogistika wie beispielsweise Kortikoiden oder Desinfizienzien kombiniert werden.

Die anti-mykotische Wirkung des erfindungsgemäßen Mittels wurde in Kulturen mit verschiedenen Malassezia-Spezies nachgewiesen. Diese Malassezia-Hefen wurden unter aeroben Bedingungen auf selektivem Agar für pathogene Pilze (Selektivagar=SA, Merck Darmstadt) bei 32° C bebrütet, wobei der Selektivagar mit einer dünnen Schicht von kaltsterilisiertem Olivenöl überschichtet wurde. Nach 8 bis 10 Tagen wurden die Malassezia-Hefen geerntet und in Ethyl-Caprylat (octanoic acid ethyl ester, CAS 106-32-1, Sigma-Aldrich, St. Luis) suspendiert. Nach der Bestimmung der Zellzahl mit einer Neubauer-Zählkammer wurde mit Xylen als Verdünnungsmedium eine Suspension mit einer Konzentration von 10⁵ Malassezia-Hefen pro µl hergestellt. Petrischalen mit Selektivagar wurden mit dieser Suspension beimpft. Dem verwendeten Selektivagar wurden die erfindungsgemäßen Alkali- oder Erdalkalisalze, nämlich Lithiumsuccinat und Calciumsuccinat in Konzentrationen von 2, 4 und 8 % (w/v) beigefügt. Die so präparierten Petrischalen mit Selektivagar, Malassezia-Suspension und den erfindungsgemäßen Alkali- oder Erdalkalisalzen wurden bei 32° C für 0, 1, 3, 6, 24, 48 und 72 Stunden inkubiert. Anschließend wurde die Malassezia-Suspension mit Chloroform (CHCl₃) aus den Petrischalen entfernt und in gasdichte Röhrchen überführt.

Von diesen Proben wurde eine High Performance Thin-Layer Chromatografie (HPTLC) durchgeführt, um die Konzentration von Ethyl-Caprylat und der freien Oktansäure zu bestimmen, welche von den verschiedenen Malassezia-Spezies zu den verschiedenen Zeitpunkten gebildet wurden. Von jeder Probe wurden 2 µl auf HPTLC-Platten (Merck Darmstadt) mittels Camag Linomat IV (Camag, Muttenz) überführt und mittels HPTLC mit Hexan:Diethylether:Ameisensäure als Spülmedium (Konzentration: 80:20:2 v/v/v) fraktioniert. Anschließend wurden die HPTLC-Platten in eine 0,1 %-ige Lösung von Primulin (Sigma-Aldrich, St. Luis) in Methylalkohol, welches anschließend mit Aceton im Verhältnis 1:10 (v/v) verdünnt wurde, getaucht. Die Ergebnisse der HPTLC wurden densitometrisch mit einem Camag TLC Scanner 3 (Camag, Muttenz) quantifiziert, unter Verwendung der Camag Software (Camag, Muttenz). Als Standards wurden Ethyl-Caprylat und Oktansäure (Sigma-Aldrich, St. Luis) parallel getestet. Die Absolutwerte wurden durch den Abgleich mit den Standardkurven ermittelt. Die Konzentration der freien Fettsäuren (FFA) zum Zeitpunkt 0 wurde von den gemessenen Konzentrationen zu den späteren Zeitpunkten subtrahiert, um die freien Fettsäuren aus dem kaltsterilisierten Olivenöl auszuschließen.

Als Ergebnis ist festzustellen, dass die getesteten Alkali- oder Erdalkalisalze Lithium-Succinat und Calcium-Succinat die Bildung freier Fettsäuren durch die Malassezia-Hefen dosisabhängig reduzieren. Es entstand in Anwesenheit der Alkali- oder Erdalkalisalze Lithium-Succinat und Calcium-Succinat ein Niederschlag, der als entsprechende Seife identifiziert werden konnte.

Aber auch andere Kombinationen aus Alkali- und/oder Erdalkalisalzen, ausgewählt aus Lithium-, Natrium-, Kalium-, Magnesium-, Calcium-, Beryllium-, Barium- und Strontium-Succinat und/oder Lithium-, Natrium-, Kalium-, Magnesium-, Calcium-, Beryllium-, Barium- und Strontium-Gluconat zeigen eine gute Wirksamkeit gegen Malassezia-Hefen.

Bei einer Konzentration von 8 % Lithium-Succinat im Selektivagar und bei der Verwendung von Ethyl-Oleat als freies Oleat wurde das Wachstum der Malassezia-Spezies *M. sympodialis* vollständig unterbrochen, da in diesem Fall ein Mangel an verfügbaren freien Fettsäuren für die Malassezia-Hefen vorliegt.

Nachfolgend wird die Erfindung anhand von Beispielen näher erläutert, wobei die Erfindung nicht auf besagte Ausführungsformen beschränkt ist. Bei der Verwendung von Kombinationen aus Alkali- und/oder Erdalkalisalzen, ausgewählt aus Lithium-, Natrium-, Kalium-, Magnesium-, Calcium-, Beryllium-, Barium- und Strontium-Succinat und/oder Lithium-, Natrium-, Kalium-, Magnesium-, Calcium-, Beryllium-, Barium- und Strontium-Gluconat wurde das Wachstum der Malassezia-Hefen ebenfalls verringert bzw. unterbrochen.

### Ausführungsbeispiele

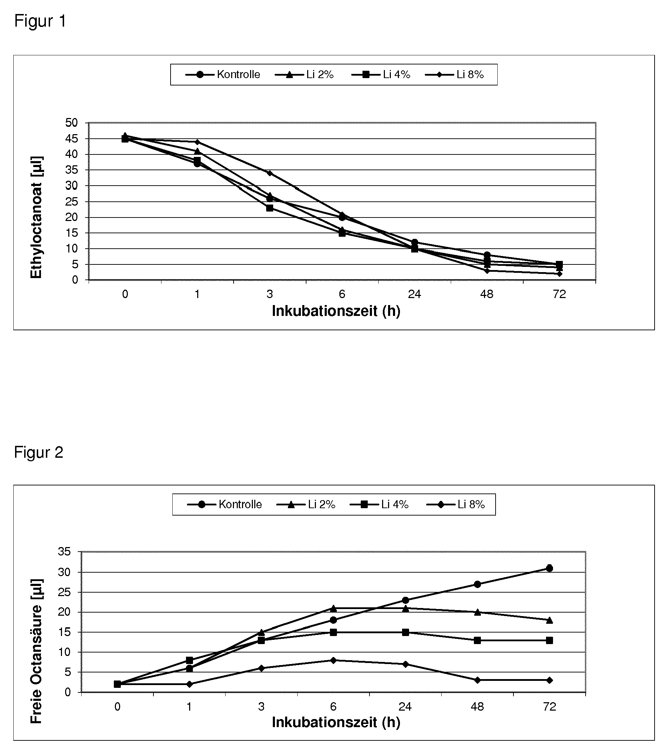

In einem Testsystem, basierend auf der Hydrolyse von Ethyloctanoat, kann die hydrolytische Aktivität von Referenzstämmen der Malassezia-Spezies *M. globosa, M. sympodialis* und *M*. *furfur* getestet werden, ohne dass der Faktor des Pilzwachstums die Ergebnisse beeinflusst. Der Grund dafür liegt in der antimykotischen Aktivität der generierten freien Oktansäure. In diesem Testsystem beeinflussen die Alkali- oder Erdalkalisalze in Konzentrationen von 2, 4 und 8 % (w/v) überraschenderweise nicht die hydrolytische Aktivität der Pilze, sondern die Verfügbarkeit der auf diesem Weg gewonnenen freien Fettsäuren. Die getesteten Alkali- oder Erdalkalisalze sind Lithium-, Natrium-, Kalium-, Calcium- und Magnesium-Succinat. Zusätzliche wurden auch die Alkali- oder Erdalkalisalze Lithium-, Natrium-, Kalium-, Calcium- und Magnesium-Gluconat auf ihre Wirksamkeit gegen Malassezia-Hefen getestet. Ebenso wurden Kombinationen dieser Alkali- oder Erdalkalisalze auf ihre Wirksamkeit gegen Malassezia-Hefen getestet. Alle diese Alkali- oder Erdalkalisalze sowie auch die Kombinationen dieser Salze reagieren mit den freien Fettsäuren zu Alkali- oder Erdalkaliseifen, somit sind diese freien Fettsäuren nicht mehr für den Stoffwechsel der Malassezia-Hefen verfügbar, wodurch das Wachstum und die Vermehrung der Malassezia-Hefen gehemmt wird (Figur 1 und 2).

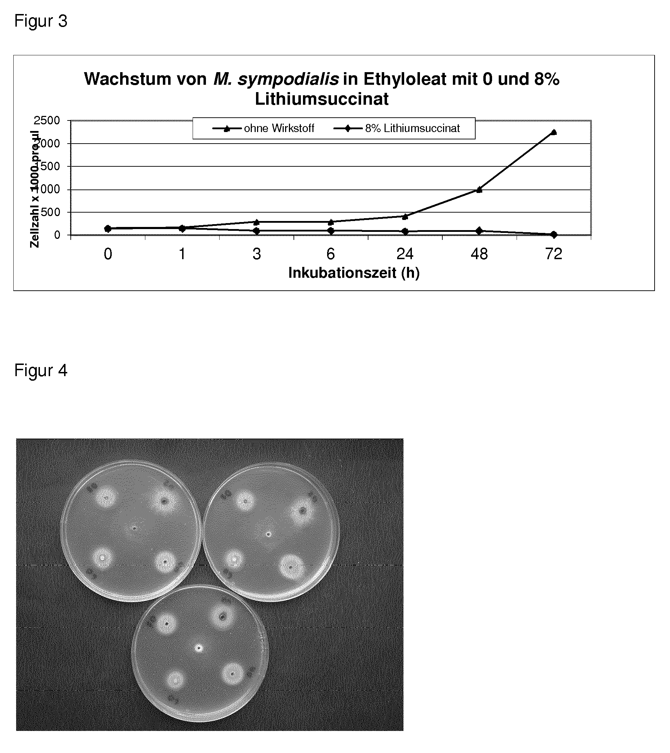

Wird in dem zuvor beschriebenen Testsystem statt Ethyloctanoat Ethyloleat eingesetzt, so kann durch die hydrolytische Aktivität der Malassezia-Hefen freie Ölsäure generiert werden, die assimilierbar ist und zu exponentiellem Wachstum der Hefen führt. Bei Anwesenheit der oben genannten Alkalisalze in den erwähnten Konzentrationen bleibt das Wachstum aus (Figur 3).

In einem Plattendiffusionstest in Form eines Lochtestes (Auxanogramm) konnte gezeigt werden, dass die Einbringung der jeweiligen Alkaliseifen in einem Stanzloch zu keinem Wachstumshof führt. Dies zeigt, dass die Alkaliseifen nicht verstoffwechselt werden können (Figur 4) und deshalb kein Wachstum der Malassezia-Hefen rund um das zentrale Loch stattfindet, welches mit den Alkaliseifen Lithium-Oleat, Natrium-Oleat bzw. Kalium-Oleat (1:10 v/v in Aqua dest.) gefüllt wurden.

Das erfindungsgemäße Mittel enthält Alkali- oder Erdalkalisalze in einer Konzentration von 0,005 bis 10 % (g/v), bevorzugt in einer Konzentration von 4 bis 8 % (g/v).

Die Alkali- oder Erdalkalisalze werden in der wässrigen Phase des erfindungsgemäßen Mittels gelöst. Die Alkali- oder Erdalkalisalze sind in dieser wässrigen Lösung des erfindungsgemäßen Mittels stabil.

Die bevorzugte pharmazeutische Zubereitungsform der Alkali- oder Erdalkalisalze im erfindungsgemäßen Mittel sind Öl-Wasser- bzw. Wasser-Öl-Gemische oder Emulsionen. Ein Beispiel dafür ist folgende Salbenzusammensetzung: 1 g Salbe als erfindungsgemäßes Mittel enthält als Wirkstoff 80 mg Lithium-Succinat, 0,50 mg Zinksulfat-7-Wasser, sowie als Hilfsstoffe Lithium-Hydroxid-1-Wasser, Polyglyceryl-4-Isostearat, dünnflüssiges Paraffin, Hartparaffin, gereinigtes Wasser, weißes Vaselin und Citronensäure-Monohydrat. Dieses Beispiel ist nicht abschließend, dem Fachmann sind auch andere Salbenzusammensetzungen und Zusammensetzungen für weitere topische Verabreichungsmittel bekannt.

Weitere Bestandteile die bei der Formulierung des erfindungsgemäßen Mittels verwendet werden, umfassen z.B. folgende pharmazeutisch unschädliche Exzipientien und/oder Additive:
- (thixotrope) hydrophile Konsistenzgeber wie Methyl-, Hydroxypropylcellulose, Xanthangummi, Polyacrylsäuren, Stärke, Gelatine, Alginate, Kieselsäure, Magnesiumaluminiumsilikat, Bentonit;
- (thixotrope) lipophile Konsistenzgeber wie Wollwachs, Glycerolmonostearat, Cetylpalmitat, Cetylstearylalkohol, Bienenwachs und Hartparaffin;
- Verdünnungsmittel bzw. Dispersionsmittel wie Wasser, einwertige Alkohole wie Ethanol, Isopropanol oder deren Mischungen und mehrwertige Alkohole wie Glycole;
- fette Öle wie Sojaöl, Kokosnussöl, Erdnussöl, Avocadoöl, Nachtkerzenöl, Olivenöl, Baumwollsaatöl, Saffloröl;
- Ethyloleat, Isopropylmyristat oder Oleyloleat;
- Siliconöle wie Dimethicon;
- flüssige oder halbfeste Kohlenwasserstoffe wie Vaseline;
- Hartparaffin, Ceresin, Mineralöle bzw. Paraffinöle oder niedrigschmelzende Wachse;
- Solubilisierungsmittel;
- Stabilisierungsmittel;
- Puffer, Emulgatoren oder Tenside, wie Fettsäureester wie z.B. Laureth-4, Steareth-2, Ceteareth-12, Oleth-5 oder Sorbitanfettsäureester wie z.B. Sorbitanmonolaurat, -stearat, oder Polysorbat 20, 40 oder 60;
- Polyglycerylester, Zuckerester oder Rizinusölverbindungen;
- Benetzungsmittel, Feuchthaltemittel wie Glycerin, Propylenglycol, Hexylenglycol, Butandiol und Sorbitol;
- Fettsäuren/Fettalkohole;
- Farbstoffe, Konservierungsmittel wie Benzoesäure, Sorbinsäure, Pheoxyethanol, Benzylalkohol, Parabene und Benzalkoniumchlorid;
- Antioxidantien wie Alphatocopherol, Ascorbinsäure, Butylhydroxytoloul, Propyl-, Dodecylgallat sowie Synergisten wie Citronensäure oder EDTA;
- Feststoffe wie Talkum, Titan(IV)oxid, Mais-, Reisstärke oder hochdisperses Siliciumdioxid;
- Neutralisationsmittel wie Natronlauge, Triethanolamin oder Trometamol;
- Filmbildner wie Polyvidon oder PVP-Copolymere und Penetrationsförderer wie Diethylenglycolmonoethylether.

### Erläuterungen zu den Figuren:

**Figur 1** zeigt die Hydrolyse von Ethyloctanoat durch *Malassezia sympodialis* (CBS 7222) ohne bzw. mit 2, 4 und 8% Lithium-Succinat; (*x̅* zweier unabhängiger Messwerte). Die Kinetik der Hydrolyse des Esters durch die malassezia-eigenen Hydrolasen ist unter allen gewählten Bedingungen identisch.
**Figur 2** zeigt korrespondierend zu Figur 1 die Bildung freier Oktansäure durch *Malassezia sympodialis* (CBS 7222) ohne bzw. mit 2, 4, und 8% Lithium-Succinat (*x̅* zweier unabhängiger Messwerte). Es wird deutlich, dass die für die Malassezia-Hefe verfügbare Menge an freier Fettsäure mit zunehmender Konzentration des Lithium-Succinats abnimmt. Dies beruht auf einer Reaktion des Lithiums mit der generierten freien Fettsäure zu einer Lithiumseife.
**Figur 3** zeigt das Wachstum von *Malassezia sympodialis* (CBS 7222) in Ethyloleat ohne und mit Zusatz von 8% Lithium-Succinat zum Nährboden. Bei der Hydrolyse von Ethyloleat entsteht freie Ölsäure, welche für *Malassezia sympodialis* verwertbar ist und zu Wachstum führt. 8% Lithium-Succinat im Nährboden führt zu einer Präzipitation der Ölsäure als Lithiumseife. Die Ölsäure ist nicht mehr verwertbar, das Wachstum der Malassezia-Hefe bleibt aus.
**Figur 4** zeigt ein Tween Auxanogramm für *Malassezia furfur* (CBS 7019). Die Tweens 20, 40, 60 und 80 sind außen im Uhrzeigersinn angeordnet, in der Mitte wurde die jeweilige Alkaliseife gelöst in Aqua dest. (1:10 v/v) pipettiert (links oben Lithium-Oleat, rechts oben Natrium-Oleat und unten Kalium-Oleat. Das Wachstum nach 10 Tagen Inkubation wird durch einen Wachstumshof um das Stanzloch angezeigt. Der Wachstumshof findet sich bei allen Tweens, nicht jedoch zentral bei den jeweiligen Alkaliseifen. Das zeigt, dass die Malassezia-Hefen bei Anwesenheit der Alkali- oder Erdalkalisalze nicht wachsen.

## Patentansprüche

1. Mittel umfassend mindestens ein Alkali- und/oder Erdalkalisalz zur Verringerung der Keimzahl von Malassezia-Hefen auf der Haut bei Menschen und Tieren.

2. Mittel nach Anspruch 1, **dadurch gekennzeichnet, dass** es sich bei dem Alkali- und/oder Erdalkalisalz um Lithium-, Natrium-, Kalium-, Magnesium-, Calcium-, Beryllium-, Barium-, Strontium-Succinat und/oder Lithium-, Natrium-, Kalium-, Magnesium-, Calcium-, Beryllium-, Barium-, Strontium-Gluconat und/oder Lithium-, Natrium-, Kalium-, Magnesium-, Calcium-, Beryllium-, Barium-, Strontium-Citrat und/oder Lithium-, Natrium-, Kalium-, Magnesium-, Calcium-, Beryllium-, Barium-, Strontium-Malat handelt.

3. Mittel nach Anspruch 1 und 2, **dadurch gekennzeichnet, dass** die Konzentration des Alkali- oder Erdalkalisalzes 0,25 bis 10 % (w/v) beträgt.

4. Mittel gemäß der Ansprüche 1 bis 3, **dadurch gekennzeichnet, dass** es zusätzlich pharmazeutisch unschädliche Exzipientien und/oder Additive umfasst.

5. Verwendung eines Mittels gemäß der Ansprüche 1 bis 4 zur Prophylaxe und Therapie von Erkrankungen und kosmetischen Problemen bei Menschen und Tieren, welche unter Beteiligung von Malassezia-Hefen verursacht oder aufrechterhalten werden.

6. Verwendung eines Mittels gemäß der Ansprüche 1 bis 4 in Kombination mit anderen Antimykotika, juckreizstillenden Substanzen, Antiphlogistika oder Desinfizienzien.
